# EUROPÄISCHE PATENTSCHRIFT

(11) **EP 0 994 688 B1**
(45) Veröffentlichungstag und Bekanntmachung des Hinweises auf die Patenterteilung: **15.01.2003**
(21) Anmeldenummer: 99920736.8
(22) Anmeldetag: 22.04.1999
(51) Int. Cl.: A61F 13/15, A61F 13/58

(54) **HYGIENEARTIKEL ZUM EINMALIGEN GEBRAUCH**
DISPOSABLE HYGIENE ARTICLE
ARTICLE D'HYGIENE A USAGE UNIQUE

(30) Priorität: 05.05.1998 DE 19819951
(43) Veröffentlichungstag der Anmeldung: 26.04.2000
(73) Patentinhaber: Paul Hartmann Aktiengesellschaft, 89522 Heidenheim (DE)
(72) Erfinder: HALBAUER, Peter, D-89547 Gerstetten (DE)
(74) Vertreter: Dreiss, Fuhlendorf, Steimle & Becker
(86) Internationale Anmeldenummer: EP9902705
(87) Internationale Veröffentlichungsnummer: WO99056684

(56) Entgegenhaltungen:
- EP-A- 0 605 013
- EP-A- 0 755 665
- DE-U- 29 711 430
- US-A- 5 629 063

## Beschreibung

Die Erfindung betrifft einen Hygieneartikel zum einmaligen Gebrauch, insbesondere Windel, Inkontinenzprodukt oder Inkontinenzeinlage, mit einem der Bauchpartie eines Benutzers zugewandten Vorderbereich, einem der Rückenpartie des Benutzers zugewandten Rückbereich und einem dazwischen liegenden im angelegten Zustand des Artikels im Schrittbereich eines Benutzers zu liegen kommenden Mittelbereich, die eine die körperabgewandte Außenseite bildende, zumindest abschnittsweise flüssigkeitsdichte Schicht und eine die körperzugewandte Innenseite bildende, zumindest im Bereich eines darunter liegenden Saugkörpers flüssigkeitsdurchlässige Schicht aufweisen, und mit einem Verschlusssystem zum Verbinden des Rückbereichs mit dem Vorderbereich im an den Benutzer angelegten Zustand des Artikels, wobei das Verschlusssystem an seitlichen ersten Befestigungsabschnitten des Rückbereichs oder Vorderbereichs hakenbildende, mechanisch wirkende und von einer Grundfläche vorspringende erste Befestigungselemente umfasst, die mit zweiten schlaufenbildenden, mechanisch wirkenden Befestigungselementen an einem zweiten Befestigungsabschnitt auf der körperabgewandten Seite des Vorderbereichs bzw. Rückbereichs eine lösbare Halteverbindung eingehend zusammenwirken, und wobei die ersten Befestigungselemente an ihrem von der Grundfläche abgewandten freien Ende eine Haftkleberbeschichtung aufweisen, um den Artikel nach Gebrauch zum Zwecke der Entsorgung in einer zusammengerollten oder - gefalteten Konfiguration fixieren zu können, indem die ersten Befestigungselemente an der die Außenseite bildenden Schicht an Bereichen außerhalb des zweiten Befestigungsabschnitts angehaftet werden.

Ein derartiger Hygieneartikel ist aus der DE 196 54 052 C1 oder aus der entsprechenden DE-A-297 11 430 U1 bekannt.

Aus US-A-5,629,063 ist ein Hygieneartikel mit einem klebend wirkenden Verschlusssystem mit klebenden Befetigungselementen bekannt. Dort ist eine Coronabehandlung der Außenseite des Hygieneartikels erwähnt; wobei jedoch ansich eine Reduzierung der Haftkraft der klebenden Befestigungselemente erreicht werden soll.

EP-A-0 605 013 zeigt einen Hygieneartikel mit einem nur mechanisch wirkenden Verschlusssystem und erwähnt ohne weitergehende Erläuterung, dass sowohl die Innenseite als auch die Außenseite des Backsheets coronabehandelt werden könne. Eine Anhaftbarkeit der bandförmigen und sehr langen Verschlusslaschen an anderen Bereichen als speziell vorgesehenen Auftreffbereichen ist nicht beschrieben.

Ausgehend von DE 196 54 052 C1 oder der entsprechenden DE-A-297 11 430 U1 liegt der vorliegenden Erfindung die Aufgabe zugrunde, den vorstehend beschriebenen Hygieneartikel im Hinblick auf die Entsorgungsfunktion des Befestigungssystems weiter zu verbessern, d.h. sicherzustellen, dass die mechanisch wirkenden ersten Befestigungselemente mit ihrem Haftkleberauftrag an ihrem von der Grundfläche abgewandten freien Ende auf der Außenseite des zusammengerollten oder - gefalteten Artikels hinreichend haften, um zu vermeiden, dass der gebrauchte Artikel sich beim Entsorgen öffnet. Es soll also sichergestellt werden, dass die ersten Befestigungsabschnitte mit den mechanisch wirkenden ersten Befestigungselementen nicht nur im ersten Moment auf der Außenseite des zusammengerollten Artikels haften, um den Artikel päckchenförmig in ein Abfallbehältnis geben zu können, sondern der Artikel soll auch nach einiger Zeit noch in dieser Konfiguration verbleiben, um zu verhindern, dass unangenehme Gerüche aus einem für die Aufnahme derartiger Artikel vorgesehenen Behältnis in einem Kinderzimmer oder Krankenzimmer wahrgenommen werden müssen. Gerade im zusammengerollten Zustand eines gebrauchten Artikels wirken erhebliche Scherkräfte, welche die ersten Befestigungsabschnitte von ihrem Auftreffbereich auf der Außenseite des zusammengerollten Artikels abzulösen suchen.

Diese Aufgabe wird bei einem Hygieneartikel der genannten Art erfindungsgemäß dadurch gelöst, dass die die körperabgewandte Außenseite bildende Schicht eine Folienschicht ist und dass die Folienschicht an ihrer Außenseite zumindest im Rückund/oder Mittelbereich des Artikels corona-behandelt ist und dort eine gegenüber dem unbehandelten Zustand oder gegenüber unbehandelten Bereichen der Folienschicht größere Benetzbarkeit im Bereich zwischen 35 und 50 dyn/cm aufweist und dadurch eine stärkere Anhaftung der ersten Befestigungsmittel bewirkt wird.

Wenn vorstehend von einer Benetzbarkeit im Bereich zwischen 35 und 50 dyn/cm gesprochen wird, so wird diese Benetzbarkeit nach der Prüfungsmethode gemäß der DIN-Norm Nr. 53364 vom Juni 1975 ermittelt. Diese Norm beschreibt eine Prüfung der Benetzbarkeit von Polyethylen- und Polypropylen-Folien durch Flüssigkeiten. Es wird die Oberflächenspannung in dyn/cm derjenigen Prüfungsflüssigkeit als Benetzbarkeit der Folie angegeben, welche die zu untersuchende Folienoberfläche gerade noch benetzt.

Mit der Erfindung wurde festgestellt, dass durch die Einwirkung elektrischer Entladung in Form einer Corona-Behandlung der Außenseite des Rück- und/oder Mittelbereichs der äußeren Folienschicht des Hygieneartikels eine Erhöhung der Haftkraft der ersten mechanisch wirkenden mit einem Haftkleberauftrag versehenen Befestigungselemente erreicht werden kann, so dass eine zuverlässige Entsorgungsfunktion des Verschlusssystems des Artikels sichergestellt werden kann.

Das Corona-Behandeln von Kunststoff-Folienmaterialien ist ansich bekannt; hierdurch soll üblicherweise die Bedruckbarkeit von Kunststofffolien erhöht werden. Dies wurde auch bereits auf dem einschlägigen Fachgebiet eingesetzt, so beschreibt die US-Patentschrift 5,629,063 eine Folienschicht Corona-zu-behandeln, um eine schwach haftende Beschichtung (release coating) als Auftreffbereich für eine Befestigungslasche auf die Folienschicht aufdrucken zu können. Ziel dieser Vorbehandlung war es, eine dauerhafte Anhaftung der aufgedruckten schwach haftenden Beschichtung zu erreichen, da anderenfalls in unerwünschter Weise die Haftkraft der Befestigungslasche auf dem Auftreffbereich wieder erhöht werden würde.

Die US-Patentschrift 5,106,383 lehrt, ein als Auftreffbereich für einen Befestigungsabschnitt dienendes Frontal-Tape, welches als Rollenware bereitgestellt wird und daher eine Haftverminderungsschicht trägt (Release-Beschichtung), einer Corona-Behandlung zu unterziehen, damit die reduzierte Klebekraft der Haftverminderungsschicht wieder erhöht wird.

Hinweise auf den Einsatz einer Corona-Behandlung bei mechanischen Verschlusssystemen oder zur Verbesserung der Entsorgungsfunktion lassen sich diesen Druckschriften nicht entnehmen.

In Weiterbildung der Erfindung wird vorgeschlagen, durch Corona-Behandlung der Folienschicht eine Benetzbarkeit zwischen 40 und 45 dyn/cm einzustellen (wiederum bestimmt mit der Prüfungsmethode nach DIN 53 364).

Die Anhaftung oder Haftkraft der ersten Befestigungsabschnitte wird auf noch im Einzelnen zu beschreibende Weise als Abschälkraft (Peel-off-Kraft) und auch als Haft-Scherkraft bestimmt.

Die auch als Peel-off-Kräfte bezeichnenden Abschälkräfte werden in der nachstehend beschriebenen Weise definiert und gemessen. Hierfür wird ein 25 mm breiter und 20 mm langer Teststreifen eines Materials, welches einen Befestigungsabschnitt, beispielsweise eine Verschlusslasche, mit Verschlusselementen der ersten Art bildet, zur Verfügung gestellt. Dieser Teststreifen wird unter einem Anpressdruck von 2 kg unter Verwendung eines Überrollgeräts auf die zu testende Gegenfläche aufgebracht. Hierbei handelt es sich um einen Abschnitt der die Außenseite bildenden Folienschicht des Artikels, auf welche (bei Gebrauch) die ersten Befestigungsabschnitte im zusammengerollten oder -gefalteten Zustand des Artikels zum Entsorgen des Artikels haftend aufgebracht werden. Dieser Abschnitt der Folienschicht wird auf einen starren Halter aufgebracht. Der starre Halter wird in einem Zugprüfgerät fixiert, und der Teststreifen wird an einer Zugbacke geklemmt, so dass sich ein Abzugswinkel von 150° ergibt, der sich beim Abziehen geringfügigst um einige wenige Grad verringert. Unter Messung der Abschälkraft (Peel-off-Kraft) wird der Teststreifen, also der erste die mechanisch wirkenden Befestigungselemente der ersten Art aufweisende Befestigungsabschnitt, von der Gegenfläche in Richtung seiner Länge (20 mm) mit einer konstanten Geschwindigkeit von 300 mm/min abgeschält. Die gemessene Abschälkraft wird dabei als Funktion des Wegs aufgezeichnet. Hierbei können - wie üblicherweise bei Abschälversuchen - Kraftspitzen, d.h. mehrere Kraftmaxima, auftreten. Die im folgenden angegebenen Werte für die Abschälkraft wurden solchenfalls als Mittelwerte (Mediane analog DIN 53 539) bestimmt.

In besonders bevorzugter Weise liegt die Abschälkraft, mit der die ersten Befestigungselemente im angehafteten Zustand auf Corona-behandelten Bereichen der Folienschicht von dieser abziehbar sind, zwischen 1 und 2 N/25mm, vorzugsweise zwischen 1,4 und 1,8 N/25mm.

Es kann auch eine Haft-Scherkraft ermittelt werden, mit der die Befestigungselemente der ersten Art auf der Folienschicht anhaftbar sind. Diese Haft-Scherkraft wird in der vorstehend beschriebenen Weise gemessen, wobei jedoch der Abzugswinkel 0° beträgt. Die Abzugskraft wirkt also quasi in der Ebene der Folienschicht, etwa vergleichbar derjenigen Kraft, welche einen zusammengerollten gebrauchten Artikel wieder zu öffnen sucht. - Der hierfür verwandte Teststreifen wurde - um den vorherigen Gebrauch des Verschlusssystems bei einem Hygieneartikel zu simulieren - zunächst dem an erster Stelle beschriebenen Abschältest unterzogen, wobei der Teststreifen jedoch auf den zweiten Befestigungsabschnitt mit Befestigungselementen der zweiten Art, nämlich auf ein flauschförmiges Material in Form einer gewirkten Velourware, wie sie von der Firma Köster unter der Bezeichnung "Loop Frontal Tape FT 600 T" erhalten werden kann, aufgebracht und danach wieder abgezogen wurde. Der Teststreifen wurde also zuvor - wie beim wirklichen Gebrauch - als Primärverschlusssystem verwandt und danach zum Simulieren der Entsorgungsfunktion auf die Folienschicht aufgebracht, um die Haft-Scherkraft zu ermitteln. Die geometrische Abmessung des Teststreifens betrug für die Ermittlung der Haft-Scherkraft wiederum 25 mm Breite und 20 mm Länge. Es hat sich als besonders vorteilhaft erwiesen, wenn die Haft-Scherkraft zwischen 10 und 12 N liegt.

In besonders bevorzugter Weise umfasst der Hygieneartikel mechanische Befestigungselemente der ersten Art, die wie in den Ansprüchen 6 bis 13 beschrieben ausgebildet sind, da sie einerseits eine zuverlässige Verbindung der mechanischen Befestigungselemente der ersten und zweiten Art beim Tragen des Hygieneartikels sicherstellen, indem mechanisch wirkende Befestigungselemente zum Einsatz kommen, die gegenüber Verschmutzung unempfindlich sind, und andererseits dennoch eine zuverlässige Fixierung des gebrauchten Artikels im zusammengerollten oder -gefalteten Zustand zum Entsorgen des Artikels gewährleistet ist.

Weitere Merkmale, Einzelheiten und Vorteile der Erfindung ergeben sich aus den beigefügten Schutzansprüchen sowie der zeichnerischen Darstellung und nachfolgenden Beschreibung einer bevorzugten Ausführungsform des erfindungsgemäßen Hygieneprodukts. In der Zeichnung zeigt:
- Figur 1: eine Ansicht eines erfindungsgemäßen Hygieneartikels in Form einer Windel;
- Figur 2: eine Schnittansicht mit der Schnittebene II-II in Figur 1;
- Figur 3: eine perspektivische Ansicht einer Windel in an einen Benutzer angelegtem Zustand;
- Figur 4: eine schematische Schnittansicht eines ersten Befestigungsabschnitts der Windel mit der Schnittebene IV-IV in Figur 1;
- Figur 5: eine vergrößerte Darstellung eines mechanisch wirkenden Befestigungselements des ersten Befestigungsabschnitts nach Figur 4;
- Figur 6: eine vergrößerte Darstellung einer zweiten Ausführungsform eines mechanisch wirkenden Befestigungselements; und
- Figur 7: eine Schnittansicht eines zweiten Befestigungsabschnitts der Windel mit der Schnittebene VII-VII in Figur 3.

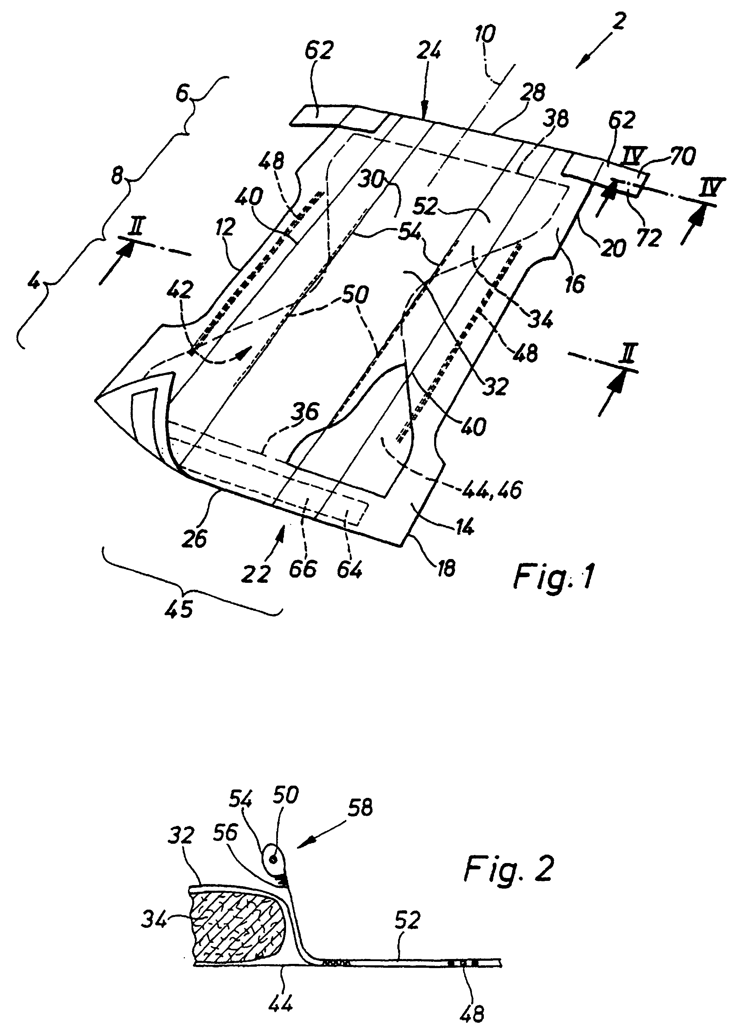

Figur 1 zeigt einen Hygieneartikel in Form einer Windel 2 für ein Kleinkind oder für inkontinente Personen. Die Windel 2 wird im Schrittbereich zwischen den Beinen eines Benutzers angeordnet und dann nach oben in Richtung auf die Hüften des Benutzers an den Körper angelegt und auf noch näher zu beschreibende Weise so fixiert, dass eine Art Höschen gebildet wird.

Die Windel besteht aus einem Vorderbereich 4, einem Rückbereich 6 und einem dazwischen liegenden, im Schrittbereich des Benutzers der Windel zu liegen kommenden Mittelbereich 8. Der Vorderbereich 4, der Mittelbereich 8 und der Rückbereich 6 sind in einer Längsrichtung 10 angeordnet. Der Mittelbereich weist zwei in Längsrichtung 10 verlaufende Längsränder 12 auf, die zum Vorderbereich 4 und zum Rückbereich 6 hin gekrümmt nach außen verlaufen, um in Seitenklappen 14, 16 am Vorderbereich 4 bzw. am Rückbereich 6 überzugehen. Die Seitenklappen 14, 16 erstrecken sich mit ihren Längsrändern 18, 20 im wesentlichen wieder parallel zur Längsrichtung 10, was jedoch nicht zwingend erforderlich ist. Der Vorderbereich 4 und der Rückbereich 6 weisen desweiteren eine quer zur Längsrichtung 10 verlaufende, einen Bundendabschnitt 22 bzw. 24, der im angelegten Zustand der Windel 2 im Hüftbereich des Benutzers zu liegen kommt, begrenzenden Querrand 26, 28 auf. Der jeweilige Längsrand 12 der Windel 2 mit seinem zu den Seitenklappen 14, 16 nach außen gekrümmten Verlauf definiert im angelegten Zustand der Windel eine jeweilige Beinöffnung.

Eine die körperzugewandte Seite der Windel 2 bildende innere Oberfläche 30 ist von einer flüssigkeitsdurchlässigen Oberflächenlage 32 gebildet, die sich in Längsrichtung vom Querrand 26 bis zum Querrand 28 erstreckt. Unterhalb der flüssigkeitsdurchlässigen Oberflächenlage 32 ist ein Saugkörper 34 vorgesehen, der - wie aus Figur 1 ersichtlich - eine sanduhrförmige Umfangsform aufweist und sich von einer Querkante 36 im Vorderbereich 4 bis zu einer Querkante 38 im Rückbereich 6 erstreckt, wobei die Querkanten 36, 38 einen Abstand in der Größenordnung von cm zum jeweiligen Querrand 26 bzw. 28 aufweisen. Der Saugkörper 34 ist also in Längsrichtung 10 vollständig von der oberen flüssigkeitsdurchlässigen Oberflächenlage 32 abgedeckt. Die Oberflächenlage 32 weist zwei gerade Längsränder 40 auf, die einen solchen Abstand voneinander aufweisen, dass sie den Saugkörper 34 im Mittelbereich 8 quer zur Längsrichtung 10 hin übergreifen, wohingegen im Vorderbereich 4 sowie im Rückbereich 6, ungefähr im Bereich der Seitenklappen 14, 16, der sanduhrförmige Saugkörper 34 unter der Oberflächenlage quer zur Längsrichtung 10 hervorragt.

Eine die körperabgewandte Außenseite 42 der Windel 2 bildende Schicht 44 ist von einer zumindest in einem zentralen Längsbereich 45 flüssigkeitsdichten Folie 46 gebildet, welche den Saugkörper 34 von außen begrenzt. Um die Erstreckung und Positionierung des Saugkörpers 34 zu fixieren, ist die obere flüssigkeitsdurchlässige Lage 32 entlang der quer verlaufenden Bundendabschnitte 22, 24 und entlang derjenigen Abschnitte der Längsränder 40, welche den Saugkörper 34 quer zur Längsrichtung 10 überlappen, mit der äußeren flüssigkeitsdichten Folie 46 verbunden, wobei hierfür ansich beliebige Verbindungen mittels Kleber oder durch Heißsiegeln oder dergleichen in Frage kommen.

Wie aus der Figur 1 desweiteren ersichtlich ist, erstrecken sich in einem Abstand zu den Längsrändern 12 und in Längsrichtung 10 erste äußere und zweite innere ansich bekannte Elastifizierungsmittel 48 bzw. 50, die äußere bzw. innere Beinabschlüsse bilden. Die äußeren und inneren Elastifizierungsmittel sind von einer hydrophoben Deckschicht 52 überfangen, die sich entlang der Längskante 12 der Windel von einem Querrand 26 zum anderen Querrand 28 erstreckt. Quer zur Längsrichtung 10 reicht die Deckschicht 52 von außen nach innen bis zu dem inneren Elastifizierungsmittel 50 und bildet dort unter Aufnahme bzw. Einschluss des inneren Elastifizierungsmittels 50 eine Falte 54, indem ein innerer längsverlaufender Randabschnitt 56 der Deckschicht 52 umgefaltet und gegen die untere Seite der Deckschicht 52 geklebt ist (Figur 2). Im Mittelbereich 8 der Windel 2 wird unter der Vorspannung des inneren Elastifizierungsmittels 50 auf diese Weise ein in Richtung auf die Körperoberfläche eines Benutzers aufstehendes Bündchen 58 gebildet. Dieses im Bereich beider Beinabschlüsse vorgesehene Bündchen 58 erstreckt sich bis in den Vorderbereich 4 und in den Rückbereich 6, um dort in Längsrichtung 10 auf Höhe der Seitenklappen 14, 16 durch Umlegen der inneren Seite der Falte 54 bzw. des Bündchens 58 gegen die innere Oberfläche 30 der flüssigkeitsdurchlässigen Oberflächenlage 32 festgeklebt, insbesondere dort aufgesiegelt zu werden. Das innere Elastifizierungsmittel 50 endet in Längsrichtung in einem Abstand von den Querrändern 26, 28 der Windel 2 und ist im Bereich der umgelegten und festgelegten Falte 54 ebenfalls fixiert, um so in Längsrichtung eine Spannung ausüben zu können, damit die Bündchen 58 "aufstehen". Die äußeren Elastifizierungsmittel 48 sind unter Längsspannung stehend zwischen der Deckschicht 52 und der flüssigkeitsundurchlässigen außenseitigen Folie 46 festgeklebt, insbesondere gesiegelt. Auch sie enden in Längsrichtung 10 in einem Abstand von den Querrändern 26, 28, was jedoch nicht zwingend notwendig ist, aber einen spannungsfreien Bundendabschnitt 22 bzw. 24 schafft.

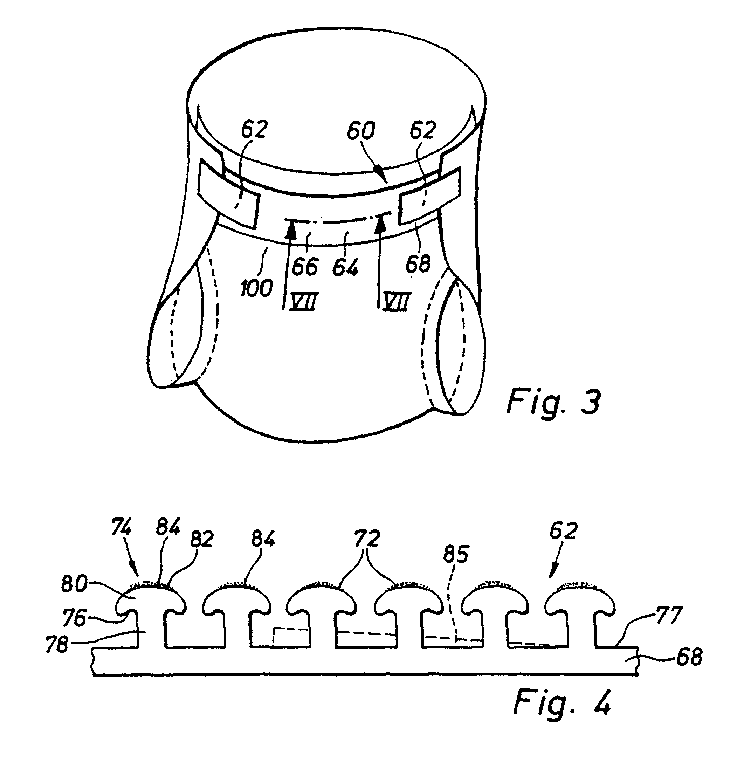

Zum Schließen der Windel im an den Benutzer angelegten Zustand ist ein insgesamt mit dem Bezugszeichen 60 bezeichnetes Verschlusssystem vorgesehen (Figur 3). Das Verschlusssystem 60 umfasst einen ersten Befestigungsabschnitt 62 an beiden Seiten des Rückbereichs 6 und einen zweiten eine Art Zielfläche für den ersten Befestigungsabschnitt 62 bildenden Befestigungsabschnitt 64, der in Form eines bandförmigen noch näher zu beschreibenden Streifens 66 an der Außenseite des Vorderteils 4 angebracht ist.

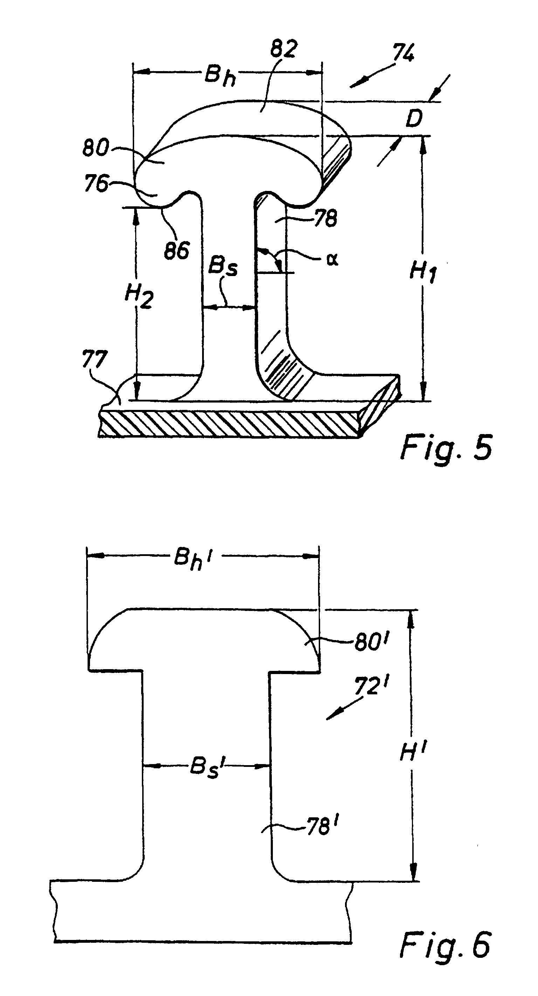

Der jeweilige Befestigungsabschnitt 62 ist von einer bandförmigen Lasche 68 gebildet. Die Lasche 68 ist ungefähr Y-förmig ausgebildet und übergreift vom Längsrand 20 der jeweiligen Seitenklappe 16 die Oberseite bzw. Unterseite der Windel 2 und ist dort festgeklebt. Die Lasche 68 weist auf ihrer im nach außen geklappten Zustand (siehe Figur 1) körperzugewandten Seite 70 mechanisch wirkende Befestigungselemente 72 auf (Figur 4, die eine schematische Schnittansicht eines die Befestigungselemente 72 aufweisenden Abschnitts der Lasche 68 zeigt). Die mechanisch wirkenden Befestigungselemente 72 sind von in der Schnittansicht T- oder pilzförmigen, hakenbildenden Vorsprüngen 74 mit hintergreifenden oder hintergreifbaren Bereichen 76 versehen. Ein einzelner derartiger Vorsprung 74 ist vergrößert in Figur 5 dargestellt. Ein solcher Vorsprung umfasst einen von einer Grundfläche 77 der Lasche 68 ausgehenden Stamm 78 und einen das freie Ende bildenden Kopf 80. Die mechanischen Befestigungselemente 72 bzw. Vorsprünge 74 weisen auf einem Teil ihrer Oberfläche 82 oder auf der gesamten Oberfläche 82 einen Klebstoffauftrag 84 in Form eines eine permanente Haftkraft aufweisenden Haftklebers auf (in Figur 5 nicht dargestellt). Anstelle eines Klebstoffauftrags können die Befestigungselemente 72 bzw. Vorsprünge 74 auch ganz aus einem permanentklebende Eigenschaften aufweisenden Material gebildet sein.

Es wäre auch denkbar, dass im Bereich zwischen den Vorsprüngen 74 auf der Grundfläche 77 ein weiterer Klebstoffauftrag 85 vorgesehen ist, wodurch die Klebekraft weiter erhöht werden kann, wenn die Lasche 68 mit großer Kraft auf einen Bereich aufgedrückt wird, zu dem der Kleber eine haftende Wirkung zeigt. Es kann sich dann - wie in der Figur 4 angedeutet - als vorteilhaft erweisen, wenn die Dicke des weiteren Klebstoffauftrags quer zur Längsrichtung 10, also in Richtung auf das freie Laschenende hin, abnimmt, so dass beim Lösen der Lasche 68 das freie Laschenende leichter gelöst werden kann.

Wie Figur 5 zu entnehmen ist, umfassen die Vorsprünge 74 eine Gesamthöhe H1 von 0,61 mm, wobei eine Distanz H2 zwischen einer Unterkante 86 des hintergreifbaren Bereichs 76 und der Grundfläche 77 der Lasche 68 0,37 mm beträgt. Die Dicke D des Stamms 78 und des Kopfs 80 beträgt 0,3 mm. Die Breite Bs des Stamms beträgt im dargestellten Fall 0,21 mm und die Breite Bh des Kopfs 80 beträgt 0,55 mm. Der Winkel a, den der Stamm 78 mit der Oberfläche 70 einschließt, beträgt 90°. Nach einem bevorzugten Ausführungsbeispiel werden 87 Vorsprünge pro cm² der Grundfläche 77 der Lasche 68 vorgesehen.

Figur 6 zeigt eine bevorzugte Ausführungsform eines pilzförmigen Vorsprungs 74' oder 72' mit kreisförmigem Querschnitt von Kopf und Stamm, dessen Kopf 80' eine Breite Bh' von 0,4 mm gegenüber einer Breite des Stamms 78' Bs' von 0,25 mm aufweist. Die Höhe des Kopfs H' gegenüber der Grundfläche beträgt 0,53 mm. Die bevorzugte Anzahl der Vorsprünge 74' beträgt 140 pro cm² Grundfläche.

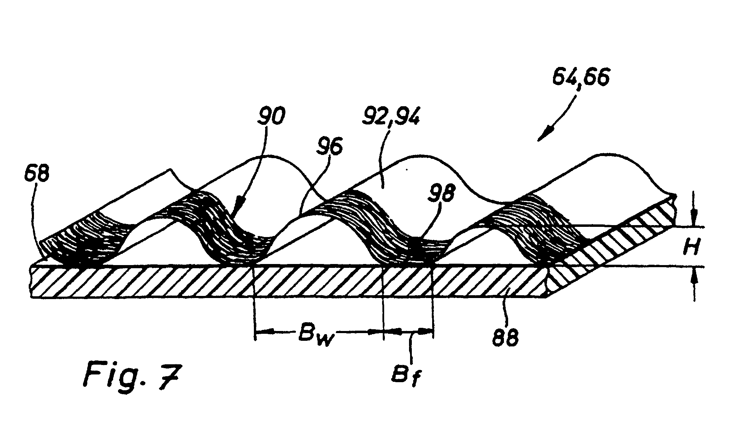

Der im Zusammenhang mit Figur 3 erwähnte erste Befestigungsabschnitt 62 wird zum Schließen der Windel 2 auf den zweiten Befestigungsabschnitt 64, also auf den Streifen 66 im Vorderbereich 4 aufgedrückt. Figur 7 zeigt eine Schnittansicht des Streifens 66. Dieser weist eine zweischichtige Struktur mit einer Trägerschicht 88 und einer zweite Befestigungselemente 90 bildenden Schlaufenschicht 92 aus einem flauschförmigen, faserigen Material 94 auf. Bei der Trägerschicht kann es sich vorzugsweise um eine Folie, ein Spinnvlies oder ein Meltblown-Vlies handeln. Die Schlaufenschicht 92 kann in vorteilhafter Weise von einem Kardenvlies oder einem Spinnvlies gebildet sein, in den die Befestigungselemente 72 des ersten Befestigungsabschnitts 62 eingreifen und verhaken können. Die Schlaufenschicht 92 weist hierfür schlaufenbildende Erhebungen 96 auf, die dadurch hergestellt sind, dass das auf die Trägerschicht 88 aufgebrachte, vorzugsweise ein Vlies bildende Nonwoven-Material 94 abschnittsweise gegen die Trägerschicht 88 fixiert ist (Bezugszeichen 98). Die Fixierung kann entlang paralleler Linien - wie in Figur 8 dargestellt - oder in Form von rauten-, dreieck- oder kreisförmigen Mustern durch einen Heißsiegelprozess erfolgen.

Für die Haltekraft ist die Ausführung der Schlaufenschicht 92 von großer Bedeutung. Bei dem in Figur 7 dargestellten Ausführungsbeispiel bilden die Erhebungen 96 endlose Wellen, wobei deren Breite Bw 2 bis 5 mm und die Breite der fixierten Fläche 98 quer zu deren Erstreckung Bf 0,2 bis 5 mm beträgt. Die Höhe H der Erhebungen 96 beträgt vorzugsweise 0,2 bis 5 mm. Es wird vorzugsweise eine Faserstärke des flauschförmigen Materials 94 von 6 bis 9 dtex und eine Faserlänge von 4 bis 5 cm gewählt. Das Flächengewicht des Materials 92 beträgt 40 bis 70 g/m². Wenn zum Schließen der Windel die Lasche 68 in korrekter Weise an dem Streifen 66 positioniert und leicht angedrückt wird, so greifen die Vorsprünge 74 in die Schlaufenschicht 92 ein und verhaken dort mechanisch.

Wenn die benutzte Windel 2 zum Entsorgen zusammengerollt oder gefaltet wird, so kann ein Benutzer die ersten Befestigungsabschnitte 62 bzw. Laschen 68 irgendwo auf die Außenseite - es wird sich im zusammengerollten Zustand der Windel dann der Mittelbereich 8 oder Rückbereich 6 als Zielfläche darstellen - aufdrücken.

Unter Verwendung eines Verschlusssystems mit einem ersten Befestigungsabschnitt mit ersten Befestigungselementen, wie sie in Figur 6 dargestellt sind, wurden Vergleichsversuche durchgeführt.

Ein Material gemäß Figur 6 ist unter der Bezeichnung CS 200, 900 XMH-4123 von der Firma 3M Deutschland erhältlich. Dieses Material ist mit 18 g/m² eines Heißschmelzhaftklebers beschichtet, welcher von der Firma Fuller, Deutschland, unter der Bezeichnung Typ 4151 erhältlich ist. Die Abmessungen der Materialabschnitte, mit denen Messungen durchgeführt wurden, betrugen 20 mm Länge und 25 mm Breite.

Die aus Polyethylen bestehende und die Außenseite einer Windel bildende Folienschicht wurde einer Corona-Behandlung unterzogen, und zwar derart, dass eine Benetzbarkeit der Folie bestimmt nach DIN 53 364 von 41 bzw. 44 dyn/cm erhalten wird. Durch eine mehr oder wenige starke Corona-Behandlung könnten auch andere Werte der Bentzbarkeit eingestellt werden. Im Vergleich hierzu lässt sich der nachfolgenden Tabelle 1 eine Benetzbarkeit von lediglich 30 dyn/cm der unbehandelten Polyethylen-Folie entnehmen.

**Tabelle 1 :**

| | unbehandelt | Corona-behandelt | |
|---|---|---|---|
| Benetzbarkeit der Folie dyn/cm | 30 | 41 | 44 |
| Abschälkräfte Haken/Folie N/25mm | 0,48 | 1,54 | 1,68 |
| Haft-Scherkräfte Haken/Folie nach Gebrauch, 10 min/20°C N | 9,1 | 10,9 | 11,1 |
| | | | |
| Brucharbeit Nm | 0,04 | 0,18 | 0,18 |
| Haft-Scherkräfte Haken/Folie nach Gebrauch, 4h/38°C, N | 9,6 | 10,6 | 11,4 |
| | | | |
| Brucharbeit Nm | 0,04 | 0,18 | 0,2 |

Es wurden mit der so behandelten Folienschicht Messungen der Abschälkraft sowie der Haft-Scherkraft des vorstehend erwähnten Materials mit mechanisch wirkenden Befestigungselementen nach der eingangs erläuterten Prüfungsmethode durchgeführt. Hierbei wurden Abschälkräfte von 0,48 N/25mm auf der unbehandelten Folienschicht und von 1,54 bzw. 1,68 N/25mm bei den beiden Corona-behandelten Folienschichten gemessen werden.

Bei der Messung der Haft-Scherkräfte, die ebenfalls genau nach der eingangs beschriebenen Prüfungsmethode durchgeführt wurde, wurde zur Simulation des Gebrauchs ein Flauschmaterial in Form einer Velour-Wirkware erhältlich unter der Bezeichnung "Loop Frontal Tape FT 600 T" von der Fa. Koester, Deutschland, als zweiter Befestigungsabschnitt verwandt. Bei der Messung nach Zeile 3 der Tabelle 1 wurde eine Haftzeit des Teststreifens auf dem Flauschmaterial von 10 min bei 20 °C gewählt; hiernach wurde der Teststreifen abgezogen. Bei der Messung der Haft-Scherkraft nach Zeile 5 der Tabelle 1 wurde eine Haftzeit von vier Stunden bei 38 °C gewählt, um dem tatsächlichen Gebrauch noch näher kommende Bedingungen zu schaffen.

Die bei einer jeweiligen Messung der Haft-Scherkräfte ermittelte Brucharbeit ist aus den Zeilen 4 und 6 der Tabelle 1 ersichtlich.

Man erkennt den positiven Einfluss, den eine Corona-Behandlung der die Außenseite bildenden Folienschicht auf die Haftkräfte der ersten Befestigungsabschnitte auf der Folienschicht beim Entsorgen des zusammengerollten Artikels hat. Die Abschälkräfte werden auf etwa das dreifache erhöht, und die Haft-Scherkräfte können um nahezu 20 % erhöht werden.

## Patentansprüche

1. Hygieneartikel zum einmaligen Gebrauch, insbesondere Windel, Inkontinenzprodukt oder Inkontinenzeinlage,
- mit einem der Bauchpartie eines Benutzers zugewandten Vorderbereich (4), einem der Rückenpartie des Benutzers zugewandten Rückbereich (6) und einem dazwischen liegenden im angelegten Zustand des Artikels im Schrittbereich eines Benutzers zu liegen kommenden Mittelbereich (8), die eine die körperabgewandte Außenseite bildende, zumindest abschnittsweise flüssigkeitsdichte Schicht (44) und eine die körperzugewandte Innenseite bildende, zumindest im Bereich eines darunter liegenden Saugkörpers (34) flüssigkeitsdurchlässige Schicht (32) aufweisen, und
- mit einem Verschlusssystem (60) zum Verbinden des Rückbereichs (6) mit dem Vorderbereich (4) im an den Benutzer angelegten Zustand des Artikels,
wobei das Verschlusssystem (60) an seitlichen ersten Befestigungsabschnitten (62) des Rückbereichs (6) oder Vorderbereichs (4) hakenbildende, mechanisch wirkende und von einer Grundfläche (77) vorspringende erste Befestigungselemente (72) umfasst, die mit zweiten schlaufenbildenden, mechanisch wirkenden Befestigungselementen (90) an einem zweiten Befestigungsabschnitt (64) auf der körperabgewandten Seite des Vorderbereichs (4) bzw. Rückbereichs (6) eine lösbare Halteverbindung eingehend zusammenwirken, und wobei die ersten Befestigungselemente (72) an ihrem von der Grundfläche (77) abgewandten freien Ende eine Haftkleberbeschichtung aufweisen, um den Artikel nach Gebrauch zum Zwecke der Entsorgung in einer zusammengerollten oder -gefalteten Konfiguration fixieren zu können, indem die ersten Befestigungselemente (72) an der die Außenseite bildenden Schicht (44) an Bereichen außerhalb des zweiten Befestigungsabschnitts (64) angehaftet werden, **dadurch gekennzeichnet, dass** die die körperabgewandte Außenseite bildende Schicht (44) eine Folienschicht ist und dass die Folienschicht an ihrer Außenseite zumindest im Rück- (6) und/oder Mittelbereich (8) des Artikels corona-behandelt ist und dort eine gegenüber dem unbehandelten Zustand oder gegenüber unbehandelten Bereichen der Folienschicht größere Benetzbarkeit im Bereich zwischen 35 und 50 dyn/cm aufweist und dadurch eine stärkere Anhaftung der ersten Befestigungsmittel (72) bewirkt wird.

2. Hygieneartikel nach Anspruch 1, **dadurch gekennzeichnet, dass** die Benetzbarkeit der Folienschicht auf der behandelten Außenseite zwischen 40 und 45 dyn/cm beträgt.

3. Hygieneartikel nach Anspruch 1 oder 2, **dadurch gekennzeichnet, dass** die Abschälkraft, mit der die ersten Befestigungselemente (72) im angehafteten Zustand auf corona-behandelten Bereichen der Folienschicht von dieser abziehbar sind, zwischen 1 und 2 N/25mm liegt.

4. Hygieneartikel nach Anspruch 3, **dadurch gekennzeichnet, dass** die Abschälkraft, mit der die ersten Befestigungselemente (72) im angehafteten Zustand auf corona-behandelten Bereichen der Folienschicht von dieser abziehbar sind, zwischen 1,4 und 1,8 N/25mm liegt.

5. Hygieneartikel nach einem der vorstehenden Ansprüche, **dadurch gekennzeichnet, dass** die Haft-Scherkraft mit der ein 20 mm langer und 25 mm breiter die Befestigungselemente (72) der ersten Art aufweisender Abschnitt auf der corona-behandelten Folienschicht anhaftbar ist, zwischen 10 und 12 N liegt.

6. Hygieneartikel nach einem oder mehreren der vorstehenden Ansprüche, **dadurch gekennzeichnet, dass** die ersten Befestigungsmittel (72) von einen hintergreifbaren Abschnitt (76) aufweisenden T- oder pilzförmigen Vorsprüngen (74) mit einer Erhebung (H1, H') von 0,2 bis 1,5 mm senkrecht zu der Grundfläche (77) gebildet sind.

7. Hygieneartikel nach Anspruch 6, **dadurch gekennzeichnet, dass** die T- oder pilzförmigen Vorspünge (74) einen von der Grundfläche (77) ausgehenden Stamm (78, 78') und einen an dessen freien Ende ausgebildeten Kopf (80, 80') aufweisen, und dass die Breite (Bs, Bs') des Stamms 0,2 bis 0,8 mm und die Breite (Bh, Bh') des Kopfs 0,1 bis 0,6 mm beträgt.

8. Hygieneartikel nach Anspruch 7, **dadurch gekennzeichnet, dass** eine gemeinsame Dicke (D) von Stamm (78) und Kopf (80) 0,1 bis 0,6 mm beträgt.

9. Hygieneartikel nach einem oder mehreren der vorstehenden Ansprüche, **dadurch gekennzeichnet, dass** die Dichte der ersten Befestigungselemente (72) 50 bis 400, vorzugsweise 100 bis 200 Stück je cm² beträgt.

10. Hygieneartikel nach einem oder mehreren der vorstehenden Ansprüche, **dadurch gekennzeichnet, dass** die Haftkleberbeschichtung von einem Haftkleberauftrag zwischen 10 und 25 g/m² Grundfläche (77) gebildet ist.

11. Hygieneartikel nach Anspruch 10, **dadurch gekennzeichnet, dass** die Haftkleberbeschichtung von einem Haftkleberauftrag zwischen 15 und 20 g/m² Grundfläche (77) gebildet ist.

12. Hygieneartikel nach Anspruch 10 oder 11, **dadurch gekennzeichnet, dass** der Haftkleber ein Heißschmelzkleber ist.

13. Hygieneartikel nach einem oder mehreren der vorstehenden Ansprüche, **dadurch gekennzeichnet, dass** die auf die Grundfläche (77) projizierte Fläche des Kopfs (80) der ersten Befestigungselemente (72) 0,1 bis 0,5 mm², vorzugsweise 0,1 bis 0,2 mm² beträgt.

14. Hygieneartikel nach einem oder mehreren der vorstehenden Ansprüche, **dadurch gekennzeichnet, dass** die zweiten Befestigungsmittel (90) von einem flauschförmigen faserigen Material gebildet sind.

15. Hygieneartikel nach Anspruch 14, **dadurch gekennzeichnet, dass** das flauschförmige Material eine gewirkte Velourware ist.

## Claims

1. Disposable hygiene article, in particular nappy, incontinence product or incontinence liner,
- with a front region (4) facing the stomach of a user, a back region (6) facing the back of the user and a middle region (8) located therebetween and coming to rest, in the applied state of the article, in the crotch region of a user, comprising a layer (44) which is liquid-tight, at least in portions, forming the body-remote outer side, and a layer (32) which is liquid-permeable, at least in the region of an absorbent member (34) located therebelow, forming the body-proximate inner side, and
- with a fastening system (60) for joining the back region (6) to the front region (4) when the article is placed on the user,
the fastening system (60) comprising hook-forming, mechanically acting first fastening elements (72) projecting from a base (77) on lateral first fastening portions (62) of the back region (6) or front region (4), the fastening elements (72) cooperating with second loop-forming, mechanically acting fastening elements (90) on a second fastening portion (64) on the body-remote side of the front region (4) or back region (6), so as to produce a detachable holding connection, and the first fastening elements (72) have an adhesive coating on their free end remote from the base (77) to fix the article after use in a rolled up or folded up configuration for disposal, in that the first fastening elements (72) on the layer (44) forming the outer side adhere to regions outside the second fastening portion (64), **characterised in that** the layer (44) forming the body-remote outer side is a film layer and **in that** the film layer is corona-treated at its outer side, at least in the back region (6) and/or middle region (8) of the article, and has greater wettability between 35 and 50 dyn/cm in the region there compared with the untreated state or compared with untreated regions of the film layer, and **in that** stronger adhesion of the first fastening means (72) is thus brought about.

2. Hygiene article according to claim 1, **characterised in that** the wettability of the film layer is between 40 and 45 dyn/cm on the treated outer side.

3. Hygiene article according to claim 1 or 2, **characterised in that** the peeling force with which the first fastening elements (72) adhering to corona-treated regions of the film layer can be removed therefrom is between 1 and 2 N/25 mm.

4. Hygiene article according to claim 3, **characterised in that** the peeling force with which the first fastening elements (72) adhering to corona-treated regions of the film layer can be removed therefrom is between 1.4 and 1.8 N/25 mm.

5. Hygiene article according to any of the preceding claims, **characterised in that** the adhesive shearing force with which a 20 mm long and 25 mm wide portion comprising the fastening elements (72) of the first type can be adhered to the corona-treated film layer is between 10 and 12 N.

6. Hygiene article according to any one or more of the preceding claims, **characterised in that** the first fastening means (72) are formed by T- or mushroom-shaped projections (74) comprising a portion (76) which can be engaged behind and an elevation (H1, H') of 0.2 to 1.5 mm perpendicular to the base (77).

7. Hygiene article according to claim 6, **characterised in that** the T- or mushroom-shaped projections (74) have a stem (78, 78') issuing from the base (77) and a head (80, 80') formed at the free end of the stem, and **in that** the width (Bs, Bs') of the stem is 0.2 to 0.8 mm and the width (Bh, Bh') of the head is 0.1 to 0.6 mm.

8. Hygiene article according to claim 7, **characterised in that** a joint thickness (D) of stem (78) and head (80) is 0.1 to 0.6 mm.

9. Hygiene article according to any one or more of the preceding claims, **characterised in that** the density of the first fastening element (72) is 50 to 400, preferably 100 to 200 pieces per cm².

10. Hygiene article according to any one or more of the preceding claims, **characterised in that** the coating of adhesive is formed by an application of adhesive between 10 and 25 g/m² of the base (77).

11. Hygiene article according to claim 10, **characterised in that** the coating of adhesive is formed by an application of adhesive between 15 and 20 g/m² of the base (77).

12. Hygiene article according to claim 10 or 11, **characterised in that** the adhesive is a hot-melt adhesive.

13. Hygiene article according to any one or more of the preceding claims, **characterised in that** the face of the head (80) of the first fastening element (72) projected onto the base (77) is 0.1 to 0.5 mm², preferably 0.1 to 0.2 mm².

14. Hygiene article according to any one or more of the preceding claims, **characterised in that** the second fastening means (90) is formed by a fleecy, fibrous material.

15. Hygiene article according to claim 14, **characterised in that** the fleecy material is a knitted velour item.

## Revendications

1. Article d'hygiène à usage unique, en particulier une couche, un produit pour l'incontinence ou une garniture pour l'incontinence,
- comportant une zone avant (4) qui est dirigée vers la partie abdominale de l'utilisateur, une zone arrière (6) qui est dirigée vers la partie dorsale de l'utilisateur et une zone médiane (8) qui vient se placer entre les deux autres zones, quand l'article est mis en place, dans la zone de marche d'un utilisateur, lesdites zones comportant une couche (44), qui est, au moins par sections, étanche aux liquides et qui forme la face extérieure opposée au corps, et une couche (32) qui est perméable aux liquides au moins dans la zone d'un corps absorbant (34) disposé en dessous et qui forme la face intérieure dirigée vers le corps, et
- comportant un système de fermeture (60) destiné à relier la zone arrière (6) à la zone avant (4) quand l'article est mis en place sur l'utilisateur,
dans lequel le système de fermeture (60) comprend des premiers éléments de fixation (72) qui sont disposés sur des premières sections latérales de fixation (62) de la zone arrière (6) ou de la zone avant (4), qui forment des crochets, qui exercent une action mécanique, qui font saillie hors d'une surface de base (77) et qui coopèrent, en réalisant une liaison de maintien amovible, avec des deuxièmes éléments de fixation (90) qui forment des boucles, qui exercent une action mécanique et qui sont disposés sur une deuxième section de fixation (64) qui se trouve sur la face opposée au corps de la zone avant (4) ou de la zone arrière (6); et
dans lequel les premiers éléments de fixation (72) comportent, sur leur extrémité libre opposée à la surface de base (77), un revêtement auto-adhésif pour pouvoir fixer l'article, après son usage, dans le but de l'évacuer, dans une configuration où il est enroulé ou plié sur lui-même, par le fait que les premiers éléments de fixation (72) sont accrochés sur la couche (44) qui forme la face extérieure, dans des zones qui sont à l'extérieur de la deuxième section de fixation (64),
**caractérisé en ce que** la couche (44), qui forme la face extérieure opposée au corps, est une couche de film, et **en ce que** la couche de film est traitée en corona sur sa face extérieure, au moins dans la zone arrière (6) et/ou dans la zone médiane (8) de l'article et présente à cet endroit une mouillabilité qui est plus grande par rapport à l'état non traité ou par rapport aux zones non traitées de la couche de film et qui est comprise entre 35 et 50 dyn/cm et, de ce fait, la couche de film entraîne un accrochage plus fort des premiers moyens de fixation (72).

2. Article d'hygiène selon la revendication 1, **caractérisé en ce que** la mouillabilité de la couche de film sur la face extérieure traitée est comprise entre 40 et 45 dyn/cm.

3. Article d'hygiène selon la revendication 1 ou 2, **caractérisé en ce que** la force d'arrachement avec laquelle on peut arracher les premiers éléments de fixation (72) de la couche de film, quand ils sont accrochés sur les zones traitées en corona de la couche de film, est comprise entre 1 et 2 N/25 mm.

4. Article d'hygiène selon la revendication 3, **caractérisé en ce que** la force d'arrachement avec laquelle on peut arracher les premiers éléments de fixation (72) de la couche de film, quand ils sont accrochés sur les zones traitées en corona de la couche de film, est comprise entre 1,4 et 1,8 N/25 mm.

5. Article d'hygiène selon l'une quelconque des revendications précédentes, **caractérisé en ce que** la force d'adhérence au cisaillement, avec laquelle on peut accrocher une section comportant des premiers éléments de fixation (72) du premier type de 20 mm de long et 25 mm de large sur la couche de film traitée en corona, est comprise entre 10 et 12 N.

6. Article d'hygiène selon l'une quelconque ou plusieurs des revendications précédentes, **caractérisé en ce que** les premiers éléments de fixation (72) sont constitués de parties en saillie (74) qui sont en forme de « T » ou de champignon, qui comportent une section (76) pouvant être saisie par l'arrière et une bosse (H1, H') de 0,2 à 1,5 mm qui est perpendiculaire à la surface de base (77).

7. Article d'hygiène selon la revendication 6, **caractérisé en ce que** les parties en saillie (74) qui sont en forme de « T » ou de champignon comportent un tronc (78, 78') qui part de la surface de base (77) et une tête (80, 80') qui est formée sur l'extrémité libre du tronc (78, 78'), **en ce que** la largeur (Bs, Bs') du tronc est comprise entre 0,2 et 0,8 mm, et **en ce que** la largeur (Bh, Bh') de la tête est comprise entre 0,1 et 0,6 mm.

8. Article d'hygiène selon la revendication 7, **caractérisé en ce qu'**une épaisseur commune (D) du tronc (78) et de la tête (80) est comprise entre 0,1 et 0,6 mm.

9. Article d'hygiène selon l'une quelconque ou plusieurs des revendications précédentes, **caractérisé en ce que** la densité des premiers éléments de fixation (72) est comprise entre 50 et 400, de préférence entre 100 et 200 pièces par cm².

10. Article d'hygiène selon l'une quelconque ou plusieurs des revendications précédentes, **caractérisé en ce que** le revêtement à auto-adhérence est constitué par une couche auto-adhésive comprise entre 10 et 25 g/m² de la surface de base (77).

11. Article d'hygiène selon la revendication 10, **caractérisé en ce que** le revêtement à auto-adhérence est constituée par une couche auto-adhésive comprise entre 15 et 20 g/m² de la surface de base (77).

12. Article d'hygiène selon la revendication 10 ou 11, **caractérisé en ce que** le produit auto-adhésif est une colle à matières fondues.

13. Article d'hygiène selon l'une quelconque ou plusieurs des revendications précédentes, **caractérisé en ce que** la surface de projection de la tête (80) des premiers éléments de fixation (72) sur la surface de base (77) est comprise entre 0,1 et 0,5 mm², de préférence entre 0,1 et 0,2 mm².

14. Article d'hygiène selon l'une quelconque ou plusieurs des revendications précédentes, **caractérisé en ce que** les deuxièmes éléments de fixation (90) sont constitués par une matière fibreuse en forme de molleton.

15. Article d'hygiène selon la revendication 14, **caractérisé en ce que** la matière en forme de molleton est du velours tricoté.
